# EUROPEAN PATENT SPECIFICATION

(11) **EP 4 257 953 B1**
(45) Date of publication and mention of the grant of the patent: **03.12.2025**
(21) Application number: 23166591.0
(22) Date of filing: 04.04.2023
(51) Int. Cl.: G01N 21/39, G01N 21/3504

(54) **LASER GAS ANALYZER**
LASERGASANALYSATOR
ANALYSEUR DE GAZ LASER

(30) Priority: 05.04.2022 JP 2022062873
(43) Date of publication of application: 11.10.2023
(73) Proprietor: Yokogawa Electric Corporation, Musashino-shi, Tokyo 180-8750 (JP)
(72) Inventor: NAKAMURA, Hajime, Musashino-shi (JP)
(74) Representative: Osha BWB

(56) References cited:
- EP-A1- 1 398 617
- CN-U- 202 994 654
- JP-A- H06 205 851
- RU-C1- 2 710 083
- US-A1- 2007 007 449
- YU-KAI HE ET AL: "A Novel Carbon Monoxide Detection System Based on Infrared Absorption Used in Mine", MACHINE LEARNING AND CYBERNETICS, 2006 INTERNATIONAL CONFERENCE ON, IEEE, PISCATAWAY, NJ, USA, 13 August 2006 (2006-08-13), pages 645 - 649, XP031439296, ISBN: 978-1-4244-0061-4

## Description

### BACKGROUND

### Technical Field

The present invention relates to a laser gas analyzer.

### Description of the Related Art

A laser gas analyzer irradiates a measurement target gas with a laser light in a specific wavelength range, measures an amount of light of laser light that has passed through the measurement target gas, and calculates an attenuation of the laser light in the specific wavelength range to calculate a concentration of a component contained in the measurement target gas (for example, JP 2008-134076 A).

There is a laser gas analyzer that makes determination as failure of a light receiving unit when the light receiving unit of laser light does not perform output in response to the laser light while the light receiving unit is irradiated with laser light. However, in the laser gas analyzer described in JP 2008-134076 A, a light receiving element does not output or the output becomes extremely small in a case where an obstacle, such as powder dust, that blocks an optical path for laser light is present by a certain amount or more in a measurement target gas, and it causes a possibility of determination as failure of the light receiving element. Thus, the configuration of the laser gas analyzer in JP 2008-134076 A failed to accurately determine failure of the light receiving element.

US 2007/007449 A1 describes an optical analysis device having a gastight housing, a radiation-permeable housing element, at least a first radiation source and associated first reflector and a second radiation source and associated second reflector, at least first and second detectors, and an external reflector. The radiation sources and the detectors are located within the housing, an absorption space being formed between the external reflector and the radiation-permeable housing element. A measurement beam emitted by the first radiation source and the first reflector, after reflection on the external reflector, re-enters the housing. A reference beam is emitted by the second radiation source and the second reflector. The measurement beam, after crossing the absorption space, is guided from the measurement beam reflector directly onto the first detector and the second detector and the reference beam from the second radiation source is directly incident on the first detector and the second detector.

RU 2 710 083 C1 describes an infrared optical gas analyzer including a housing, a cuvette, two sources of infrared radiation, an amplifier with a switch, a microcontroller, infrared radiation sources power supply switches, radiation sources current limiter and an infrared radiation sensor. The cuvette is made in the form of a cylindrical tube in which the main source of infrared radiation with a reflector, an additional source of infrared radiation and an infrared radiation sensor are placed in series on the same optical axis. The radiation sensor combines two receivers of infrared radiation and a temperature sensor in its housing. Interference filters are installed in front of radiation receivers. Main and additional sources of infrared radiation are connected through the current limiter to the power supply source, and their activation is controlled by a microcontroller. Receivers of infrared radiation are displaced from the axis of the tube of the measuring cuvette so that an additional source of infrared radiation installed directly in front of the sensor housing does not screen the radiation of the main source of infrared radiation.

Yu-Kai He et al. : « A Novel Carbon Monoxide Detection System Based on Infrared Absorption Used in Mine", Machine Learning and Cybernetics, 2006 International Conference on, IEEE, Piscataway, NJ, USA, 13 August 2006, pages 645-649 describes a carbon monoxide detection system comprising dual light sources and four detectors compensating for power source anti-jamming, mismatch of the detectors, gas cell material's absorption, and dust's influence. In addition, an infrared carbon monoxide sensor's mathematical model is built by adopting radial basic function's neural network model, so as to dispel the influence of temperature, pressure and humidity.

CN 202 994 654 U describes a double light source four detector infrared gas sensor. According to an optical path model of the sensor, a detector which has a reference effect is concurrently added to a position of a working detector, namely, the two reference detectors are added. A reference light filtering piece is arranged in front of each reference detector. After to-be-detected gas is treated by the model, only transmittance of the to-be-detected gas is obtained, and the remaining factors can be eliminated. According to the double light source four detector infrared gas sensor, influence of gas chamber optical components, dust and other factors can be eliminated, and only one signal proportional to the square of the transmittance of the to-be-detected gas is produced, so that anti-interference ability and measurement accuracy are improved according to the design. JPH06205851 A describes a disaster prevention monitoring apparatus comprising a smoke detector and a test device for testing the light receiver of the smoke detector.

### SUMMARY

One or more embodiments are directed to a laser gas analyzer that allows accurately determining failure of a light receiving unit.

Embodiments of the present disclosure are defined in the claims and are described below.

A laser gas analyzer according to one or more embodiments includes a laser diode, a light receiving unit, a processor, and a light emitting diode (LED) and a processor. The laser diode is configured to irradiate a measurement target gas with laser light and intermittently irradiate the laser light. The light receiving unit comprises a photodiode configured to receive the laser light that has passed through the measurement target gas. Further, the laser gas analyzer comprises a light emitting diode (LED) configured to irradiate LED light such that the LED light is received by the photodiode without passing through the measurement target gas, and irradiate the LED light at a predetermined timing during a period in which the laser diode does not irradiate the laser light. The processor is configured to calculate a concentration of a component contained in the measurement target gas based on an amount of light of the laser light received by the photodiode, and determine presence of failure of the photodiode based on an output of the photodiode that receives both of the laser light and the LED light. If the laser light is not detected but the LED light is detected in the period, the processor determines that the laser diode and the light receiving unit are normally working. If the laser light is not detected and the LED light is not detected in the period, the processor determines that the laser diode is normally working but the light receiving unit is abnormally working.

Failure of the light receiving unit may be accurately determined by means of the laser gas analyzer according to one or more embodiments of the present disclosure.

According to one or more embodiments, the laser diode is configured to intermittently irradiate laser light at an interval of a predetermined section, and the LED is configured to irradiate light at a predetermined timing in the predetermined section.

In one or more embodiments, the predetermined section includes a settlement waiting section in which settlement of an output of the light receiving unit is waited for and a dark current measurement section for measuring a dark current, and the second irradiation unit is configured to irradiate light in the settlement waiting section.

In one or more embodiments, the laser gas analyzer further comprises an arithmetic unit comprised in the processor) and this arithmetic unit of the processor is configured to determine presence of failure of the photodiode.

In one or more embodiments, the laser diode is configured to intermittently irradiate laser light at an interval of a predetermined section, and the LED is configured to stop irradiating light at a predetermined timing in the predetermined section and to constantly irradiate light in another section.

In one or more embodiments, the LED is configured to change at least one of an amount of light or a wavelength and to irradiate light.

### BRIEF DESCRIPTION OF THE DRAWINGS

FIG. 1 is a block diagram of a laser gas analyzer according to one or more embodiments;
FIG. 2 is a drawing illustrating light emission timings of a laser diode and a LED of a laser gas analyzer according to one or more embodiments;
FIG. 3A is a drawing illustrating a light receiving signal waveform detected by a light receiving unit of a laser gas analyzer according to one or more embodiments;
FIG. 3B is a drawing illustrating a light receiving signal waveform detected by the light receiving unit of a laser gas analyzer according to one or more embodiments;
FIG. 3C is a drawing illustrating a light receiving signal waveform detected by the light receiving unit of a laser gas analyzer according to one or more embodiments;
FIG. 3D is a drawing illustrating a light receiving signal waveform detected by the light receiving unit of a laser gas analyzer according to one or more embodiments;
FIG. 4 is a drawing illustrating light emission timings of a laser diode and a LED of a laser gas analyzer according to one or more embodiments;
FIG. 5 is a block diagram of a laser gas analyzer according to one or more embodiments;
FIG. 6 is a block diagram of a Comparative Example of laser gas analyzer; and
FIG. 7 is a drawing illustrating a light emission timing of a laser diode of a Comparative Example of laser gas analyzer.

### DESCRIPTION OF THE EMBODIMENTS

The following will describe laser gas analyzers according to one or more embodiments of the present disclosure with reference to the drawings.

### <Example 1>

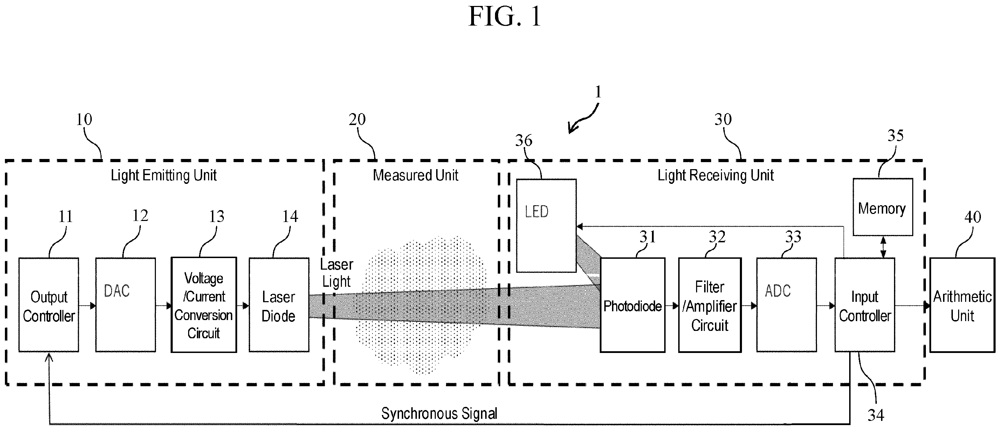

FIG. 1 is a block diagram of a laser gas analyzer according to one or more embodiments. With reference to FIG. 1, a laser gas analyzer according to one or more embodiments will be described. A laser gas analyzer 1 is a Tunable Diode Laser Absorption Spectroscopy (TDLAS) laser gas analyzer. The laser gas analyzer 1 irradiates a measurement target gas with a laser light in a specific wavelength range, measures an amount of light of laser light that has passed through the measurement target gas, and calculates a concentration of a component contained in the measurement target gas based on an attenuation at a specific wavelength. The laser gas analyzer 1 includes a light emitting unit 10 that irradiates a laser light in a specific wavelength range, a measured unit 20 to which a measurement target gas is supplied, a light receiving unit 30 that receives a laser light that has passed through the measurement target gas, and an arithmetic unit 40 that calculates a concentration of a component contained in the measurement target gas. Note that the laser gas analyzer 1 need not include the measured unit 20. For example, in a case where the light emitting unit 10 and the light receiving unit 30 are mounted for use on a facility to which the measurement target gas of a user who uses the laser gas analyzer 1 is supplied, the laser gas analyzer 1 need not include the measured unit 20. Additionally, the arithmetic unit 40 may be a computer system built into the laser gas analyzer 1 or may be a computer system (for example, a cloud server) that can communicate with the laser gas analyzer 1.

### (Light Emitting Unit 10)

The light emitting unit 10 includes an output controller 11, a Digital Analog Converter (DAC) 12, a voltage/current conversion circuit 13, and a laser diode 14. The output controller 11 generates a pattern of laser drive current in accordance with a synchronous signal from an input controller 34 in the light receiving unit 30 described later and outputs it. The DAC 12 converts the pattern (digital signal) of the laser drive current output by the output controller 11 into an analog signal and outputs it. The voltage/current conversion circuit 13 outputs the laser drive current in accordance with the analog signal output by the DAC 12. The laser diode 14 irradiates a laser light in a specific wavelength range in accordance with the laser drive current output from the voltage/current conversion circuit 13. The laser diode 14 is a first irradiation unit that irradiates the measurement target gas with laser light. The laser light irradiated by the laser diode 14 passes through the measurement target gas supplied to the measured unit 20 and is received by a photodiode 31 in the light receiving unit 30.

### (Measured Unit 20)

The measurement target gas is supplied to the measured unit 20. The measurement target gas is, for example, a fuel exhaust gas and a process gas. The laser gas analyzer 1 can calculate concentrations of components, such as O₂, CO, CH₄, CO₂, and NH₃, contained in the measurement target gas.

### (Light Receiving Unit 30)

The light receiving unit 30 includes the photodiode 31, a filter/amplifier circuit 32, an Analog Digital Converter (ADC) 33, the input controller 34, a memory 35, and an LED 36. The photodiode 31 receives the laser light that has been irradiated by the laser diode 14 and has passed through the measurement target gas supplied to the measured unit 20, and outputs an analog signal corresponding to the amount of light of the received laser light. The photodiode 31 is a light receiving unit that receives the laser light that has passed through the measurement target gas. The photodiode 31 is disposed so as to be opposed to the laser diode 14 in the light emitting unit 10 between which the measured unit 20 is interposed. The filter/amplifier circuit 32 filters a predetermined frequency component contained in the received analog signal and amplifies it. The ADC 33 converts the analog signal received from the filter/amplifier circuit 32 into a digital signal and outputs it. The input controller 34 performs primary arithmetic processing, such as integration processing, on the digital signal received from the ADC 33 and stores it in the memory 35. The digital signal stored in the memory 35 indicates a waveform of the amount of received light of the laser light that has passed through the measurement target gas. Hereinafter, the digital signal stored in the memory 35 is referred to as a light receiving signal waveform as appropriate.

The LED 36 is disposed on the photodiode 31 side with respect to the measured unit 20. The LED 36 is a second irradiation unit that irradiates light such that the light is received by the photodiode 31 without the light passing through the measurement target gas. The light irradiated by the LED 36 is received by the photodiode 31 without the light passing through the measurement target gas. Accordingly, even when an obstacle, such as powder dust, is present in the measurement target gas, the light irradiated by the LED 36 is received by the photodiode 31 without being affected by the obstacles. The input controller 34 controls a light emission timing of the LED 36.

The arithmetic unit 40 reads the light receiving signal waveform stored in the memory 35 and calculates the concentration of the component contained in the measurement target gas. The arithmetic unit 40 is a calculation unit that calculates the concentration of the component contained in the measurement target gas based on the amount of light of the laser light received by the photodiode 31. The arithmetic unit 40 is a computer, and includes a processor (for example, a Central Processing Unit (CPU), a field-programmable gate array (FPGA), or a Digital signal processor (DSP)), a memory (for example, a Random Access Memory (RAM)), an auxiliary storage unit (for example, a Hard Disk Drive (HDD) and a Solid State Drive (SSD)), an information output unit (for example, current output, contact output, or Ethernet communication) to an external device, a display unit, and the like. The processor, for example, reads a program for calculating the concentration from an auxiliary storage unit, loads it into a memory, and executes it. The display unit displays, for example, the calculated concentration, and the information output unit outputs, for example, the calculated concentration to the external device. Additionally, the processor reads, for example, a program for determination of presence of failure of the light receiving unit 30 from the auxiliary storage unit, loads it into the memory, and executes it.

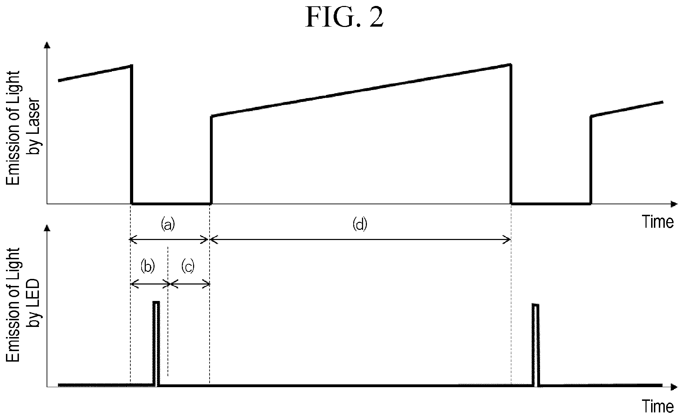

FIG. 2 is a drawing illustrating the light emission timings of a laser diode and the LED of a laser gas analyzer according to one or more embodiments. As illustrated in FIG. 2, a section (a period) (a) is a section in which laser light is not emitted, and a section (a period) (d) is a section in which laser light is emitted. The laser diode 14 intermittently irradiates the laser light at an interval of the predetermined section (section (a)). The section (a) is, for example, about 0.1 ms. The section (d) differs depending on the measurement target gas, and is, for example, in a range of about from 1 ms to 3 ms. The first half (b) in the section (a) is a settlement waiting section (a settlement waiting period) in which the settlement of the output of the photodiode 31 is waited for (i.e., paused), and the second half (c) is a dark current measurement section (a dark current measurement period) for measuring a dark current. That is, the first half (b) is the section not used for arithmetic operation by the arithmetic unit 40. The second half (c) is the section used to calculate a reference value of the arithmetic operation by the arithmetic unit 40. The settlement waiting section may be a predetermined time or may be a time until it is determined that a predetermined time has elapsed while the output of the photodiode 31 is under a threshold or less. The dark current measurement section is a predetermined time.

The LED 36 emits light in the settlement waiting section of the light receiving signal in the first half (b). The arithmetic unit 40 constantly observes presence of a spike signal by emission of light by the LED 36 in the light receiving signal waveform in the first half (b) to confirm soundness of the light receiving unit 30.

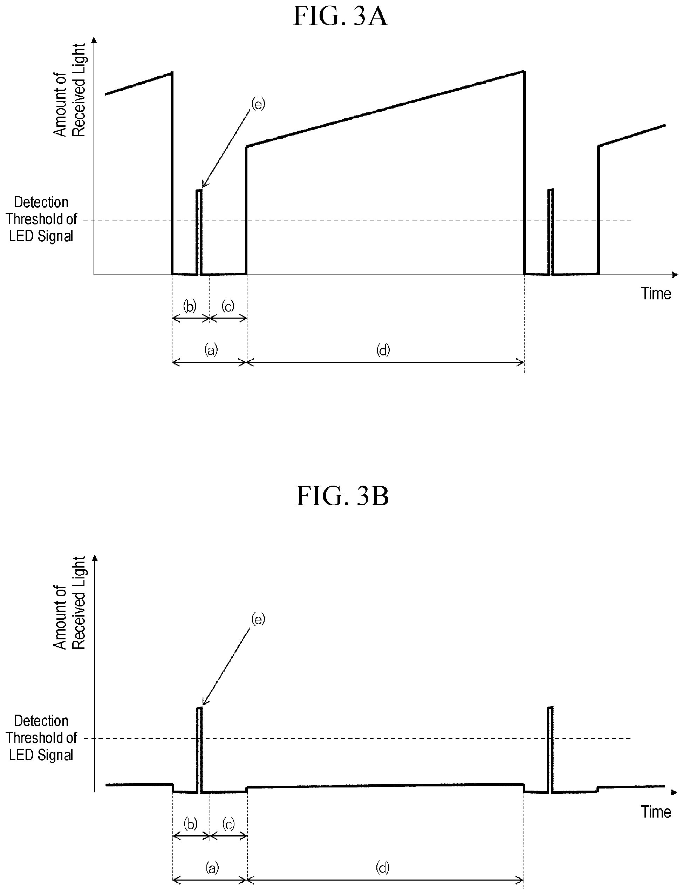

FIG. 3A to FIG. 3D are drawings illustrating the light receiving signal waveform of a laser gas analyzer according to one or more embodiments.

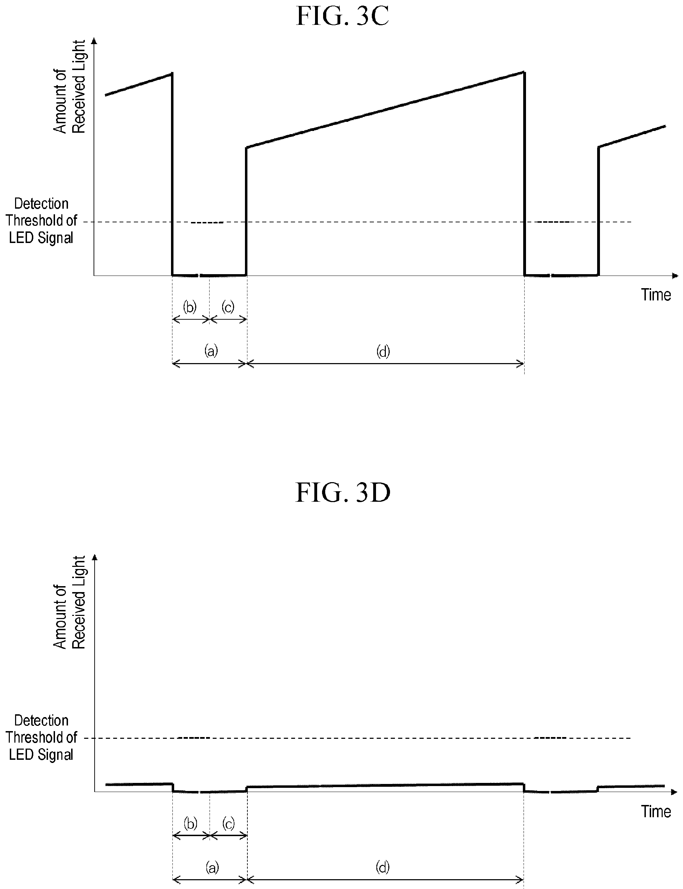

The light receiving signal waveform is divided into the following four patterns.
Pattern A: a pattern that includes both of a signal corresponding to the laser light irradiated by the laser diode 14 and a spike signal corresponding to the light irradiated by the LED 36 (see FIG. 3A)
Pattern B: a pattern that does not include the signal corresponding to the laser light irradiated by the laser diode 14 and includes the spike signal corresponding to the light irradiated by the LED 36 (see FIG. 3B)
Pattern C: a pattern that includes the signal corresponding to the laser light irradiated by the laser diode 14 but does not include the spike signal corresponding to the light irradiated by the LED 36 (see FIG. 3C)
Pattern D: a pattern that includes neither the signal corresponding to the laser light irradiated by the laser diode 14 nor the spike signal corresponding to the light irradiated by the LED 36 (see FIG. 3D)

The arithmetic unit 40 is an arithmetic unit that determines presence of failure of the photodiode 31 based on the output of the photodiode 31 that receives both of the laser light irradiated by the laser diode 14 and the light irradiated by the LED 36. When the arithmetic unit 40 has confirmed the light receiving signal waveform of Pattern A illustrated in FIG. 3A, the arithmetic unit 40 determines that both of the light emitting unit 10 and the light receiving unit 30 are normal. Additionally, when the arithmetic unit 40 has confirmed the light receiving signal waveform of Pattern B illustrated in FIG. 3B, the arithmetic unit 40 determines that both of the light emitting unit 10 and the light receiving unit 30 are normal. In this case, it is considered that while the signal corresponding to the laser light irradiated by the laser diode 14 cannot be confirmed, a certain amount or more of obstacles that block an optical path for laser light are present in the measurement target gas. Additionally, when the arithmetic unit 40 has confirmed the light receiving signal waveform of Pattern C illustrated in FIG. 3C, the arithmetic unit 40 determines that the light emitting unit 10 is normal while the LED 36 in the light receiving unit 30 is abnormal. When the arithmetic unit 40 has confirmed the light receiving signal waveform of Pattern D illustrated in FIG. 3D, the arithmetic unit 40 determines that the light emitting unit 10 is normal while at least one of the photodiode 31, the filter/amplifier circuit 32, or the ADC 33 in the light receiving unit 30 is abnormal. The display unit in the arithmetic unit 40 can display each of the determination results. Additionally, each of the determination results can be transmitted to the external device via the information output unit.

### (Effects of Example 1)

The light irradiated by the LED 36 is received by the photodiode 31 without passing through the measurement target gas. Thus, the photodiode 31 can receive the light irradiated by the LED 36 without being affected by the measurement target gas. As a result, even when a certain amount or more of obstacles are present in the measurement target gas, soundness of the light receiving unit 30 can be confirmed. As a result, reliability of failure detection is increased, leading to improvement in failure detection rate. Further, the failure detection rate is an important index in terms of functional safety, and therefore it is beneficial also in terms of functional safety standard.

The input controller 34 causes the LED 36 to emit light at the predetermined timing in the settlement waiting section (the first half (b) in FIG. 2). Accordingly, using the section not used for the arithmetic operation during the usual operation in the laser gas analyzer 1, failure of the light receiving unit 30 can be determined. That is, in one or more embodiments, without stopping the usual operation of the laser gas analyzer 1, failure of the light receiving unit 30 can be determined.

### <Example 2>

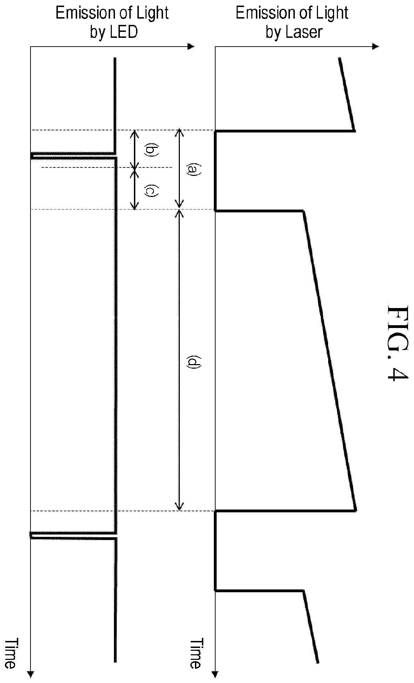

FIG. 4 is a drawing illustrating light emission timings of the laser diode and the LED of another example of a laser gas analyzer according to one or more embodiments, illustrated in Example 2. The light emission timings of the LED 36 are inverted between Example 1 and Example 2. In Example 1, the LED 36 irradiates light at the predetermined timing in the settlement waiting section (b) of the light receiving signal. In Example 2, the LED 36 turns off at the predetermined timing (stops irradiating the light during a stop period) in the settlement waiting section (b) of the light receiving signal. In Example 2, the LED 36 constantly irradiates light in the section other than the predetermined timing (the stop period). In the first half (b), the arithmetic unit 40 constantly observes whether a negative spike signal by turning off the LED 36 is present in the light receiving signal waveform to confirm soundness of the light receiving unit 30.

### (Effects of Example 2)

By constantly causing the LED 36 to emit light in the section other than the predetermined timing, an amount of offset light due to emission of light by the LED 36 is added to the amount of received light of the photodiode 31. By adjusting the amount of light of the light irradiated by the LED 36, the photodiode 31 can receive the laser light and perform output in the range where accuracy of the output with respect to the input of the photodiode 31 is high. Consequently, the photodiode 31 can output the value at high accuracy with respect to the received laser light. Since the other effects are similar to those of Example 1, the description thereof will be omitted.

### <Example 3>

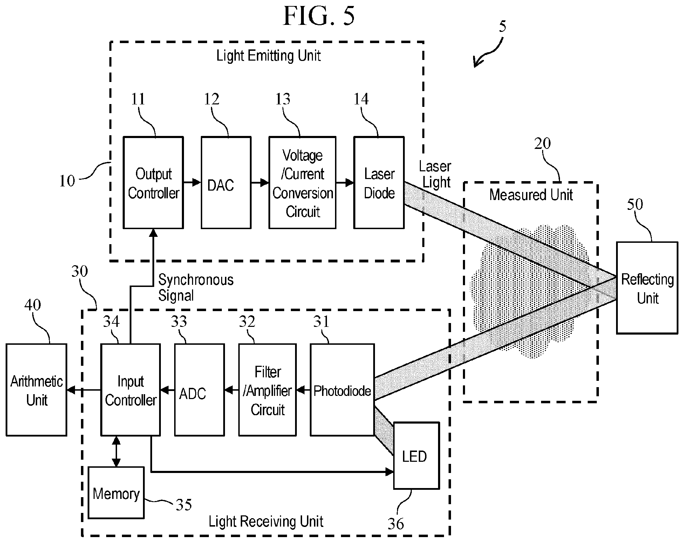

FIG. 5 is a block diagram of a laser gas analyzer of another example of a laser gas analyzer according to one or more embodiments, illustrated in Example 3. With reference to FIG. 5, a laser gas analyzer 5 of Example 3 will be described. Similarly to the laser gas analyzer 1 of Example 1, the laser gas analyzer 5 of Example 3 includes the light emitting unit 10, the measured unit 20, the light receiving unit 30, and the arithmetic unit 40. The measured unit 20 of the laser gas analyzer 5 is a probe that retrieves the measurement target gas. Additionally, the laser gas analyzer 5 includes a reflecting unit 50 that reflects laser light. The laser light irradiated by the laser diode 14 passes through a gas supplied to the measured unit 20 and is reflected by the reflecting unit 50. The reflected laser light is received by the photodiode 31. The light emitting unit 10 and the light receiving unit 30 of Example 3 are disposed on the same side with respect to the measured unit 20.

### (Effects of Example 3)

The laser gas analyzer 5 including the probe type measured unit 20 allows obtaining the effects similar to those of Example 1.

Although the disclosure has been described with respect to only a limited number of embodiments, those skilled in the art, having benefit of this disclosure, will appreciate that various other embodiments may be devised without departing from the scope of the present invention, which is determined by the claims.

For example, the number of the LEDs 36 in Examples 1 to 3 is one, but may be two or more.

Additionally, in examples not falling under the scope of the invention, the LEDs 36 in Examples 1 to 3 may be another luminous body, such as a laser diode, and may be able to change an amount of light and a wavelength of the light irradiated by the luminous body. By changing the amount of light and the wavelength of the light irradiated by the luminous body and evaluating the change in the amount of light detected by the photodiode 31, soundness and performance of the photodiode 31 can be evaluated. The above-described change in the wavelength may be achieved by a combination of turning on and turning off lights of a plurality of luminous bodies.

Additionally, when the laser gas analyzers of Examples 1 to 3 have an Auto Gain function, it is preferred that the Auto Gain function does not work on a pulse signal by the LED 36.

Moreover, when the LED 36 is constantly lit as in Example 2, use of AC coupling is preferred.

Additionally, in Examples 1 to 3, failure of the light receiving unit 30 is determined using the settlement waiting section. However, for example, it is only necessary to prepare a failure diagnosis mode for the laser gas analyzer and emit lights of the photodiode 31 and the LED 36 in the failure diagnosis mode.

### <Comparative Example>

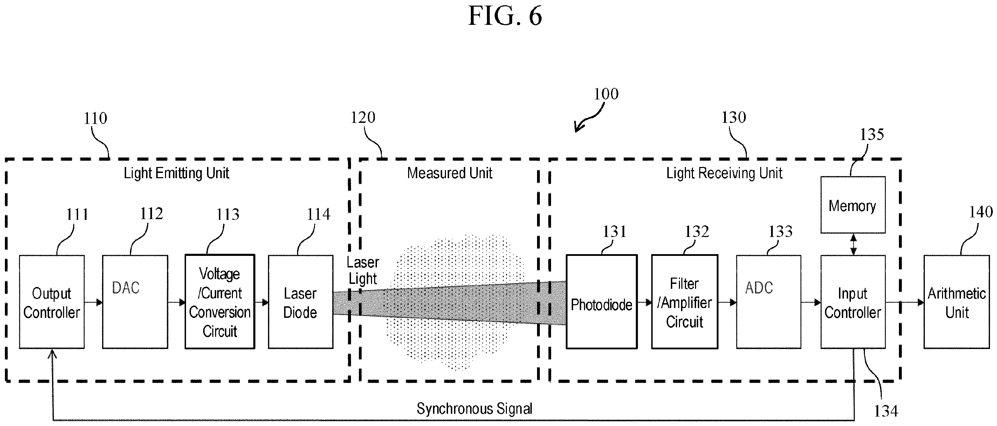

FIG. 6 is a block diagram illustrating a laser gas analyzer of Comparative Example. With reference to FIG. 6, a circuit configuration and an operation of a laser gas analyzer 100 of Comparative Example will be described.

In a light emitting unit 110, an output controller 111 receives a synchronous signal from an input controller 134 in a light receiving unit 130 and generates a pattern of laser drive current. The pattern is supplied as drive current to a laser diode 114 via a Digital Analog Converter (DAC) 112 and a voltage/current conversion circuit 113. Thus, the laser diode 114 irradiates laser light. The laser light after passing through the measurement target gas is detected by a photodiode 131 in the light receiving unit 130. The output of the photodiode 131 is input to the input controller 134 via a filter/amplifier circuit 132 and an Analog Digital Converter (ADC) 133. The input controller 134 sequentially stores the output of the photodiode 131 in a memory 135. An arithmetic unit 140 reads the output of the photodiode 131 stored in the memory 135 and calculates a concentration value.

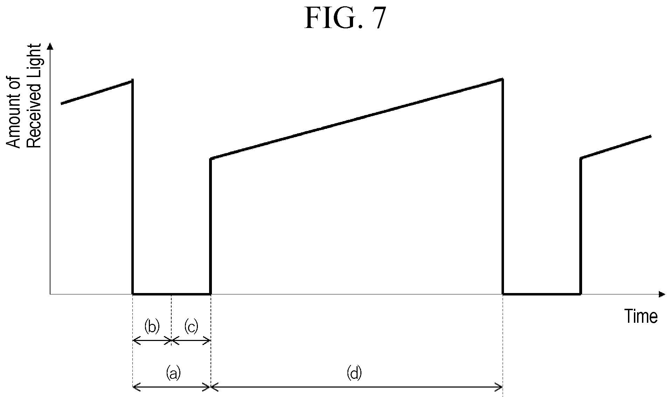

FIG. 7 is a drawing illustrating a light receiving signal waveform detected by the laser gas analyzer of Comparative Example. The light emission timing of the laser light will be described with reference to FIG. 7. In FIG. 7, a section (a) is a section in which laser light is not emitted, and a section (d) is a section in which laser light is emitted. Emission and no emission of the laser light are alternately repeated.

Next, a method for failure diagnosis of the photodiode 131, the filter/amplifier circuit 132, and the ADC 133 in the light receiving unit 130 in the laser gas analyzer 100 of Comparative Example will be described. When any of the photodiode 131, the filter/amplifier circuit 132, and the ADC 133 has failure, there is a high possibility of the output value of the ADC 133 fixed to a certain constant value. Therefore, in the laser gas analyzer 100 of Comparative Example, when the variation of the output of the ADC 133 is in a constant range, it is determined that the light receiving unit 130 (any of the photodiode 131, the filter/amplifier circuit 132, and the ADC 133) has failure.

However, not only the case where any of the photodiode 131, the filter/amplifier circuit 132, and the ADC 133 has failure, but also the case where a certain amount or more of an obstacle, such as powder dust, blocking the optical path for laser light is present in the measurement target gas, the variation of the output of the ADC 133 possibly is in a constant range. Accordingly, in the method for failure diagnosis of Comparative Example, the case where a certain amount or more of an obstacle, such as powder dust, blocking the optical path for laser light is present in the measurement target gas, it is determined as failure of the light receiving unit 130, and therefore the failure of the light receiving unit 130 was not able to be accurately determined.

### DESCRIPTION OF SYMBOLS

- 1: Laser gas analyzer
- 10: Light emitting unit
- 11: Output controller
- 12: DAC
- 13: Voltage/current conversion circuit
- 14: Laser diode
- 20: Measured unit
- 30: Light receiving unit
- 31: Photodiode
- 32: Filter/amplifier circuit
- 33: ADC
- 34: Input controller
- 35: Memory
- 36: LED
- 40: Arithmetic unit
- 50: Reflecting unit

## Claims

1. A laser gas analyzer (1) comprising:
a laser diode (14) configured to:
irradiate a measurement target gas with laser light; and
intermittently irradiate the laser light;
a light receiving unit (30) comprising a photodiode (31) configured to receive the laser light that
has passed through the measurement target gas;
a light emitting diode (36), LED, configured to:
irradiate LED (36) light such that the LED light is received by the photodiode (31) without passing through the measurement target gas; and
irradiate the LED light at a predetermined timing during a period in which the laser diode (14) does not irradiate the laser light; and a processor (40) configured to:
calculate a concentration of a component contained in the measurement target gas based on an amount of light of the laser light received by the photodiode (31); and
determine presence of failure of the photodiode (31) based on an output of the photodiode that receives both of the laser light and the LED light, wherein:
if the laser light is not detected but the LED light is detected in the period, the processor (40) determines that the laser diode (14) and the light receiving unit (30) are normally working, and
if the laser light is not detected and the LED light is not detected in the period, the processor (40) determines that the laser diode (14) is normally working but the light receiving unit (30) is abnormally working.

2. The laser gas analyzer according to claim 1, wherein
the period includes:
a settlement waiting period during which settlement of an output of the photodiode (31) is paused; and
a dark current measurement period for measuring a dark current, and
the LED (36) is configured to irradiate the LED light during the settlement waiting period.

3. The laser gas analyzer according to claim 1 or claim 2, wherein the LED (36) is configured to
change at least one of an amount of light of the LED light or a wavelength of the LED light.

## Patentansprüche

1. Lasergasanalysator (1), umfassend:
eine Laserdiode (14), die konfiguriert ist zum:
Bestrahlen eines Messungszielgases mit Laserlicht; und
intermittierendes Abstrahlen des Laserlichts;
eine Lichtempfangseinheit (30), die eine Fotodiode (31) umfasst, die konfiguriert ist zum Empfangen des Laserlichts, welches das Messungszielgas durchquert hat;
eine lichtemittierende Diode (LED) (36), die konfiguriert ist zum:
Abstrahlen des Lichts der LED (36), sodass das LED-Licht von der Fotodiode (31) empfangen wird, ohne das Messungszielgas zu durchqueren; und
Abstrahlen des LED-Lichts zu einem vorbestimmten Zeitpunkt während eines Zeitraums, in dem die Laserdiode (14) das Laserlicht nicht abstrahlt; und einen Prozessor (40), der konfiguriert ist zum:
Berechnen einer Konzentration einer Komponente, die in dem Messungszielgas enthalten ist, basierend auf einer Lichtmenge des Laserlichts, das von der Fotodiode (31) empfangen wird, und
Ermitteln eines Vorhandenseins eines Ausfalls der Fotodiode (31) basierend auf einer Ausgabe der Fotodiode, die sowohl das Laserlicht als auch das LED-Licht empfängt,
wobei:
der Prozessor (40), wenn das Laserlicht nicht erkannt wird, aber das LED-Licht in dem Zeitraum erkannt wird, ermittelt, dass die Laserdiode (14) und die Lichtempfangseinheit (30) normal funktionieren, und
der Prozessor (40), wenn das Laserlicht nicht erkannt wird und das LED-Licht nicht in dem Zeitraum erkannt wird, ermittelt, dass die Laserdiode (14) normal funktioniert, aber die Lichtempfangseinheit (30) anormal funktioniert.

2. Lasergasanalysator nach Anspruch 1, wobei:
der Zeitraum beinhaltet:
einen Abwicklungswartezeitraum, während dem eine Abwicklung einer Ausgabe der Fotodiode (31) unterbrochen ist; und
einen Dunkelstrommesszeitraum zum Messen eines Dunkelstroms, und
die LED (36) konfiguriert ist zum Abstrahlen des LED-Lichts während des Abwicklungswartezeitraums.

3. Lasergasanalysator nach Anspruch 1 oder Anspruch 2, wobei die LED (36) konfiguriert ist zum Verändern mindestens einer von einer Lichtmenge des LED-Lichts oder einer Wellenlänge des LED-Lichts.

## Revendications

1. Un analyseur de gaz laser comprenant :
une diode laser (14) configurée pour :
irradier un gaz cible de mesure avec de la lumière laser ; et
irradier la lumière laser de manière intermittente ;
une unité de réception de lumière (30) comprenant une photodiode (31) configurée pour recevoir la lumière laser qui a traversé le gaz cible de mesure ;
une diode électroluminescente (36), LED, configurée pour :
irradier de la lumière de LED (36) de sorte que la lumière de LED soit reçue par la photodiode (31) sans traverser le gaz cible de mesure ; et
irradier la lumière de LED à un moment prédéterminé pendant une période au cours de laquelle la diode laser (14) n'irradie pas la lumière laser ; et
un processeur (40) configuré pour :
calculer une concentration d'un composant présent dans le gaz cible de mesure sur la base d'une quantité de lumière de la lumière laser reçue par la photodiode (31); et
déterminer la présence d'une défaillance de la photodiode (31) sur la base d'une sortie de la photodiode qui reçoit à la fois la lumière laser et la lumière de LED,
dans lequel :
si la lumière laser n'est pas détectée mais que la lumière de LED est détectée durant la période, le processeur (40) détermine que la diode laser (14) et l'unité de réception de lumière (30) fonctionnent normalement, et
si la lumière laser n'est pas détectée et que la lumière de LED n'est pas détectée pendant la période, le processeur (40) détermine que la diode laser (14) fonctionne normalement mais que l'unité de réception de lumière (30) fonctionne de manière anormale.

2. L'analyseur de gaz laser selon la revendication 1, dans lequel
la période comprend :
une période d'attente d'établissement pendant laquelle l'établissement d'une sortie de la photodiode (31) est suspendu ; et
une période de mesure du courant d'obscurité pour mesurer un courant d'obscurité, et
la LED (36) est configurée pour irradier la lumière de LED pendant la période d'attente d'établissement.

3. L'analyseur de gaz laser selon la revendication 1 ou selon la revendication 2, dans lequel la LED (36) est configurée pour modifier au moins l'un des éléments suivants : une quantité de lumière de la lumière de LED ou la longueur d'onde de la lumière de LED.
